# EUROPEAN PATENT APPLICATION

(11) **EP 4 624 964 A1**
(43) Date of publication of application: **01.10.2025**
(21) Application number: 25164111.4
(22) Date of filing: 17.03.2025
(51) Int. Cl.: G01R 31/54

(54) **CIRCUIT MODULE, CELL PUNCTURE DEVICE, AND MICROSCOPE SYSTEM**

(30) Priority: 29.03.2024 JP 2024057956
(71) Applicant: Yokogawa Electric Corporation, Tokyo 180-8750 (JP)
(72) Inventor: KUWATA, Masahiro, Musashino-shi, Tokyo, 180-8750 (JP); YOSHIDA, Fumihiro, Musashino-shi, Tokyo, 180-8750 (JP)
(74) Representative: Stöckeler, Ferdinand

(57) **Abstract**

A circuit module 30 according to the present disclosure outputs a voltage to a circuit element 16b1, and includes a first connector 31 arranged on the circuit element 16b1's side, a second connector 32 arranged on the opposite side to the first connector 31, a cable 33 connecting between the first and second connectors 31 and 32, a first checker that changes a status when the first or second connector 31 or 32 is disconnected from the cable 33, a second checker that changes a status when the first or second connector 31 or 32 is disconnected from the cable 33, a first breaker 34 that contributes a break of the voltage in response to a change in the status of the first checker, and a second breaker 37 that contributes a break of the voltage in response to a change in the status of the second checker.

## Description

### CROSS-REFERENCE TO RELATED APPLICATION

This application claims priority to Japanese Patent Application No. 2024-057956, filed on March 29, 2024, the entire contents of which are incorporated herein by reference.

### TECHNICAL FIELD

The present disclosure relates to a circuit module, a cell puncture device, and a microscope system.

### BACKGROUND

In conventional research and applied development related to cells, technology for puncturing cells with needles is known, in order to accurately inject chemical solutions or the like into specific cells as samples, or to suck substances inside cells. For example, Patent Literature (PTL) 1 discloses that, in a device that uses a multi-barrel nano-pipette with at least two electrodes inside multiple barrels, one barrel draws out a cell inclusion and the other injects a substance into a cell.

### CITATION LIST

### Patent Literature

PTL 1: JP 6453300 B2

### SUMMARY

In conventional cell puncture devices, circuit elements such as piezoelectric elements are used to puncture cells with needles. However, safety in supplying a voltage to the circuit elements is not fully investigated even in PTL 1.

It would be helpful to provide a circuit module, a cell puncture device, and a microscope system that improve safety in supplying a voltage to a circuit element.

A circuit module according to some embodiments is a circuit module configured to output a voltage to a circuit element, the circuit module including:
a first connector arranged on the circuit element's side;
a second connector arranged on the opposite side to the first connector;
a cable connecting between the first connector and the second connector;
a first checker configured to change a status when the first or second connector and the cable are in a disconnected state;
a second checker configured to change a status when the first or second connector and the cable are in a disconnected state;
a first breaker configured to contribute a break of the voltage in response to a change in the status of the first checker; and
a second breaker configured to contribute a break of the voltage in response to a change in the status of the second checker.

This improves safety in supplying the voltage to the circuit element. Due to the provision of the first checker and the first breaker, the circuit module can contribute the break of the voltage to the circuit element at a single point when the cable is not connected to at least one of the first connector or the second connector. Due to the provision of the second checker and the second breaker, the circuit module can contribute the break of the voltage to the circuit element at another point when the cable is not connected to at least one of the first connector or the second connector. As a result, the circuit module can contribute the break of the voltage at multiple points when the cable is in the disconnected state, thus allowing more reliable suppression of output of the voltage.

In the circuit module according to one embodiment, the first checker may include first wiring that loops between the first breaker and the first connector via the second connector and the cable. This allows the circuit module to directly break the voltage to the circuit element in response to a change in the conduction status of the first wiring. Therefore, when the cable is in the disconnected state, the circuit module can suppress output of the voltage more reliably.

In the circuit module according to one embodiment, the second checker may include second wiring that loops between the second breaker and the first connector via the second connector and the cable. This allows the circuit module to indirectly break the voltage to the circuit element in response to a change in the conduction status of the second wiring. Therefore, when the cable is in the disconnected state, the circuit module can suppress output of the voltage more reliably.

In the circuit module according to one embodiment, the first checker may include:
a first communication element arranged at a first node between the cable and the first connector, the first communication element being configured to detect the disconnected state between the first connector and the cable and output a first communication signal to the first breaker; and
a second communication element arranged at a second node between the cable and the second connector, the second communication element being configured to detect the disconnected state between the second connector and the cable and output a second communication signal to the first breaker.

This allows the circuit module to directly break the voltage to the circuit element, in response to a change in the output status of the communication signal from each communication element. Therefore, when the cable is in the disconnected state, the circuit module can suppress output of the voltage more reliably.

In the circuit module according to one embodiment, the second checker may include:
a third communication element arranged at a third node between the cable and the first connector, the third communication element being configured to detect the disconnected state between the first connector and the cable and output a third communication signal to the second breaker; and
a fourth communication element arranged at a fourth node between the cable and the second connector, the fourth communication element being configured to detect the disconnected state between the second connector and the cable and output a fourth communication signal to the second breaker.

This allows the circuit module to indirectly break the voltage to the circuit element, in response to a change in the output status of the communication signal from each communication element. Therefore, when the cable is in the disconnected state, the circuit module can suppress output of the voltage more reliably.

The circuit module according to one embodiment may further include a converter arranged on the second connector's side, the converter configured to convert a low-voltage signal that is input to the circuit module into a high-voltage signal to drive the circuit element. Therefore, the circuit module can easily drive the circuit element even when a high voltage is required to drive the circuit element.

In the circuit module according to one embodiment, the first breaker may be arranged between the converter and the second connector, and may be configured to break the high-voltage signal that is output from the converter. Therefore, the circuit module can directly break, using the first breaker, the voltage to the circuit element.

In the circuit module according to one embodiment, the second breaker may be arranged between a power line from outside the circuit module and the converter, and may be configured to break power supply to the converter. Therefore, the circuit module can indirectly break, using the second breaker, the voltage to the circuit element.

In the circuit module according to one embodiment, the second breaker may be arranged between a signal line from outside the circuit module and the converter, and may be configured to break the low-voltage signal to be input to the converter. This allows the circuit module to indirectly break, using the second breaker, the voltage to the circuit element. The circuit module can suppress output of the high-voltage signal more reliably by performing double breaks using the second breaker and the first breaker when the cable is in the disconnected state, and therefore improve safety.

The circuit module according to one embodiment may further include:
a third breaker configured to contribute a break of the voltage in response to a change in the status of the first checker, and
wherein the third breaker may be arranged between a power line from outside the circuit module and the first breaker, and is configured to break power supply to the first breaker, in order to break the first breaker or cancel the break of the first breaker.

This allows the circuit module to indirectly break, using the third breaker, the voltage to the circuit element. The circuit module further has the third breaker, and therefore can break the voltage using the three breakers when the cable is in the disconnected state. The circuit module can perform double breaks using the second breaker and the third breaker at a low-voltage side to the first breaker that needs to be selected as one that can be used at a high voltage. Therefore, the circuit module can suppress output of the high-voltage signal more reliably, and improve safety.

A cell puncture device according to some embodiments includes:
the circuit module described above;
the circuit element; and
a needle configured to be driven by the circuit element, the needle configured to puncture a cell.

This improves safety in supplying the voltage to the circuit element. Due to the provision of the first checker and the first breaker, the cell puncture device can contribute the break of the voltage to the circuit element at a single point when the cable is not connected to at least one of the first connector or the second connector. Due to the provision of the second checker and the second breaker, the cell puncture device can contribute the break of the voltage to the circuit element at another point when the cable is not connected to at least one of the first connector or the second connector. As a result, the cell puncture device can contribute the break of the voltage at multiple points when the cable is in the disconnected state, thus allowing more reliable suppression of output of the voltage.

In the cell puncture device according to one embodiment, the circuit element may include a piezoelectric element. This allows the cell puncture device to move the needle at a high speed when the needle punctures the cell. Therefore, in order to inject a chemical solution into the cell or to take out a substance inside the cell by suction, the cell puncture device can easily control the position and speed of a needle tip so that the needle tip penetrates a cell wall. By combining the circuit module and the piezoelectric element, the cell puncture device can improve safety even in an applied voltage to the piezoelectric element, which may be as high as 150 V, for example.

A microscope system according to some embodiments includes the cell puncture device described above.

This improves safety in supplying the voltage to the circuit element. Due to the provision of the first checker and the first breaker, the microscope system can contribute the break of the voltage to the circuit element at a single point when the cable is not connected to at least one of the first connector or the second connector. Due to the provision of the second checker and the second breaker, the microscope system can contribute the break of the voltage to the circuit element at another point when the cable is not connected to at least one of the first connector or the second connector. As a result, the microscope system can contribute the break of the voltage at multiple points when the cable is in the disconnected state, thus allowing more reliable suppression of output of the voltage.

According to the present disclosure, it is possible to provide the circuit module, the cell puncture device, and the microscope system that improve safety in supplying the voltage to the circuit element.

### BRIEF DESCRIPTION OF THE DRAWINGS

In the accompanying drawings:
FIG. 1 is a schematic diagram illustrating an example configuration of a microscope system with a cell puncture device according to a first embodiment of the present disclosure;
FIG. 2 is a schematic diagram illustrating an example configuration of a circuit module according to the first embodiment of the present disclosure;
FIG. 3 is a schematic diagram illustrating an example configuration of a circuit module according to a second embodiment of the present disclosure;
FIG. 4 is a schematic diagram illustrating an example configuration of a circuit module according to a first variation of the present disclosure; and
FIG. 5 is a schematic diagram illustrating an example configuration of a circuit module according to a second variation of the present disclosure.

### DETAILED DESCRIPTION

The background and problems of conventional technology will be described in more detail.

In recent years, in research into biological systems and the like, there have been research into elucidating cell functions by injecting specific chemical solutions into cells and observing changes in the cells, and research into causing specific modifications to occur in specific cells by injecting, into the cells, chemical solutions to modify genes. In addition, there has also been applied development aiming at application to the production of chemical solutions or other substances. On the other hand, there have also been research into elucidating cell functions and applied development aiming at application to production, by sucking and recovering, from specific cells, some of components that constitute the cells. In the above research and applied development, it is required, for example, to accurately inject the chemical solutions into the specific cells or to accurately suck the components from the specific cells, so it is desired that cell puncture devices can accurately puncture cells with needles.

PTL 1 discloses conventional technology for a method and device of injecting a chemical solution into a cell by controlling the position of a fine needle using a piezoelectric element, puncturing the cell by a needle tip, and controlling a voltage. Similarly, conventional technology for a method and device for sucking a substance from the interior of the cell is also disclosed.

In general, cells protect their interiors by cell walls, which are located in more external parts of the cells and cover the interiors. Therefore, in order to inject a chemical solution into a cell or to take out a substance from the interior of a cell by suction, a cell puncture device needs to insert a needle into the interior of the cell by controlling the position and speed of a needle tip so that the needle tip penetrates a cell wall. For this purpose, the cell puncture device preferably moves the needle at a high speed when the needle punctures the cell.

For example, in the case of a certain animal cell with a soft cell wall, the needle can penetrate the cell wall even if the cell puncture device moves the needle at a low speed when the needle punctures the cell. On the other hand, in the case of a certain plant cell with a hard cell wall, the needle cannot penetrate the cell wall unless the cell puncture device moves the needle at a high speed when the needle punctures the cell, which causes the problem of difficulty in inserting the needle tip into the cell.

As a method of moving the needle at a high speed as described above, a method of moving the needle using a circuit element such as a piezoelectric element is known. The piezoelectric element can cause an electrostrictive effect to occur inside itself by application of a voltage, and can expand and contract the piezoelectric element itself. The expansion and contraction of the piezoelectric element can respond at a higher speed than actuators such as general motors. Therefore, with the use of the piezoelectric element, the cell puncture device can cause the needle to puncture the cell at a high speed.

However, the expansion and contraction caused by the electrostrictive effect of the piezoelectric element is minute in relation to the applied voltage. In order to obtain sufficient expansion and contraction to move the needle, it is necessary to apply a high voltage to the piezoelectric element. For example, the voltage applied to the piezoelectric element may be as high as 150 V. Therefore, in wiring from a controller, which performs control, to a driver with the piezoelectric element, when a high voltage is output from the controller with the wiring being disconnected, an operator may be exposed to the high voltage upon touching the disconnected wiring.

In order to solve the above problems, it would be helpful to provide a circuit module, a cell puncture device, and a microscope system that improve safety in supplying a voltage to a circuit element.

Embodiments of the present disclosure will be mainly described below with reference to the attached drawings. In the following description, x-, y-, and z-directions are with respect to the directions of the arrows in the drawings.

### (First Embodiment)

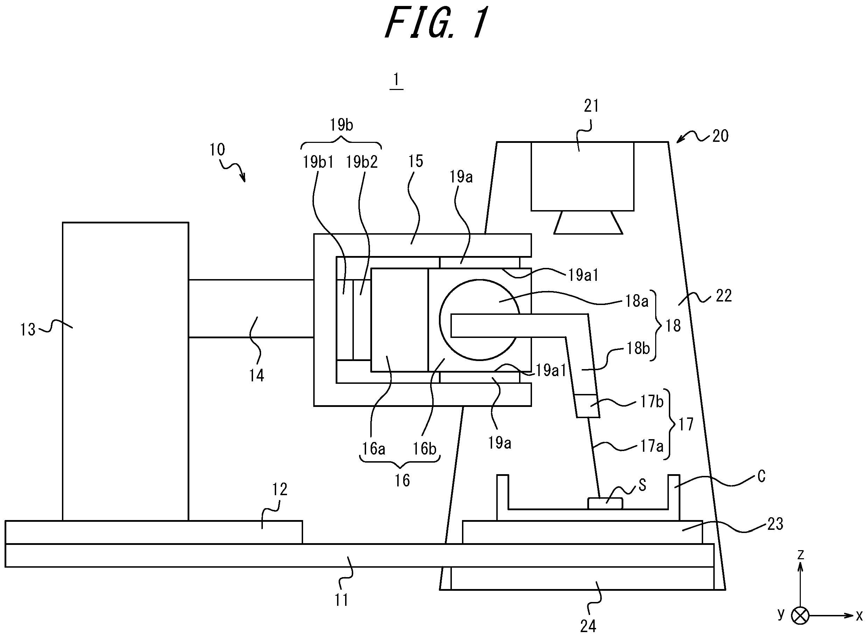

FIG. 1 is a schematic diagram illustrating an example configuration of a microscope system 1 having a cell puncture device 10 according to a first embodiment of the present disclosure. For the sake of simplifying illustration, configurations of a circuit module 30, which is described later, and the like are omitted in FIG. 1, and part of a configuration of the cell puncture device 10 is schematically illustrated. An example of the configuration and functions of the microscope system 1 having the cell puncture device 10 according to the first embodiment will be mainly described with reference to FIG. 1. The microscope system 1 has the cell puncture device 10 and a microscope 20 that images a cell S to be punctured by a needle 17a of the cell puncture device 10.

The microscope 20 includes any microscope that can image the cell S. The microscope 20 includes, for example, a confocal microscope. The microscope 20 has any camera 21 that can image the cell S. The camera 21 constitutes an imaging unit of the microscope 20. The microscope 20 has a pillar 22 that locates the camera 21 on one side in the z-direction with respect to the cell S, so that the camera 21 can image the cell S from that side. The pillar 22 supports the camera 21 connected to an end of the pillar 22 on that side. The microscope 20 has a holder 23 that holds a petri dish C, on which the cell S is disposed, from the other side in the z-direction. The holder 23 is configured as a stage that is movable in two directions, the x- and y-directions. The microscope 20 has a support 24 that is located at an end of the pillar 22 on the other side and that supports the holder 23, which holds the petri dish C.

The cell puncture device 10 has a first fixed part 11 that is arranged with respect to the imaging unit, which images the cell S. The first fixed part 11 is, for example, fixed to the microscope 20, which images the cell S. The first fixed part 11 is configured in the shape of an arm and extends in the x-direction. The first fixed part 11 is arranged with respect to the holder 23 and the support 24 and is fixed to the microscope 20, by being screwed at one side in the x-direction onto the support 24 of the microscope 20. As an example, the first fixed part 11 is located between the holder 23 and the support 24, but is not limited to this. The holder 23 may be located under the first fixed part 11. The first fixed part 11 is not limited to being screwed onto the support 24, but may be fixed to the microscope 20 in any other manner, such as by joining, fitting, or engaging. The cell puncture device 10 can be attached to the microscope 20 via the first fixed part 11.

The cell puncture device 10 is connected to the other side of the first fixed part 11 in the x-direction, and has a pedestal 12 located on a surface of the first fixed part 11. The cell puncture device 10 is supported by the first fixed part 11 and the pedestal 12, and has a first driver 13 that protrudes from the pedestal 12 to the positive side in the z-direction. The cell puncture device 10 has an arm 14 that extends from the first driver 13 to the positive side in the x-direction. The arm 14 is arranged with respect to the first fixed part 11. For example, the arm 14 is arranged in parallel with the first fixed part 11. The first driver 13 drives the arm 14 so that the arm 14 is movable in each of the x-, y-, and z-directions with respect to the pedestal 12.

The cell puncture device 10 has a support 15 that is connected to a distal end of the arm 14 in the x-direction. The support 15 is for mounting vibration dampers 19a and a regulator 19b, which are described later. The cell puncture device 10 has a second driver 16 located inside the support 15 so as to be sandwiched by an outer frame of the support 15. Not limited to this, the second driver 16 may not be located inside the support 15. The second driver 16 may be located in another place outside the support 15, together with the vibration dampers 19a. The second driver 16 is arranged with respect to the arm 14 via the regulator 19b and the support 15. The second driver 16 is arranged with respect to the support 15 via the vibration dampers 19a.

The second driver 16 needs to move the needle 17a at a high speed in order to puncture the cell S. In order to achieve such high-speed movement of the needle 17a, the second driver 16 includes, for example, a piezoelectric element that drives the needle 17a. The second driver 16 includes a second fixed part 16a that is connected to the regulator 19b, which is described later, and a first movable part 16b that is connected to the second fixed part 16a and drives the needle 17a. The first movable part 16b is movable relative to the second fixed part 16a. The piezoelectric element contained in the first movable part 16b drives the needle 17a so that a needle tip of the needle 17a moves along the z-direction. The first movable part 16b causes the needle 17a to puncture the cell S by moving relative to the second fixed part 16a.

The cell puncture device 10 has a needle unit 17 that arranges, at a tip end, the needle 17a to puncture the cell S and that is driven by the first movable part 16b of the second driver 16. The needle unit 17 has the needle 17a that is driven by the second driver 16 and punctures the cell S, and a needle fixer 17b that fixes the needle 17a.

The needle fixer 17b allows the needle 17a to be attached to and detached from a support head 18b, which is described later, when the needle 17a is replaced. The needle fixer 17b is detachably attached to the support head 18b in any manner, such as by screwing, joining, fitting, or engaging. For example, the needle fixer 17b may have a fixing screw connected to the needle 17a, and may be attached to the support head 18b by screwing the fixing screw into a screw arranged in the support head 18b. The needle fixer 17b may have, for example, an axial structure compatible with the support head 18b, and may be attached to the support head 18b by fitting with the support head 18b based on the axial structure.

The cell puncture device 10 has a needle support 18 that is connected to the needle fixer 17b and that arranges the needle unit 17 at its distal end. The needle support 18 has a mover 18a that moves the needle unit 17 between a first position in which the needle 17a punctures the cell S and a second position to which the needle 17a is evacuated. The needle support 18 has the support head 18b that couples the mover 18a and the needle fixer 17b. The mover 18a includes, as an example, a rotation mechanism to which the support head 18b, which supports the needle unit 17 at its tip end, is connected. The mover 18a may undetachably or detachably fix the support head 18b in any manner such as by screwing, joining, fitting, or engaging.

The mover 18a supports the needle unit 17 rotatably clockwise and counterclockwise around a rotation axis along the y-axis. For example, the mover 18a defines the position of the needle unit 17 illustrated in FIG. 1, as a first position in which the needle 17a punctures the cell S. For example, the mover 18a defines, as a second position, any position to which the needle unit 17 is rotatably moved counterclockwise from the first position.

The cell puncture device 10 has the vibration dampers 19a that dampen vibration of the needle 17a. The vibration dampers 19a include, for example, vibration-damping rubber. The vibration dampers 19a may be arranged with respect to at least one of the arm 14 or the second driver 16. For example, in FIG. 1, the vibration dampers 19a are arranged such that vibration-damping surfaces 19a1 contact only the first movable part 16b of the second driver 16. The pair of vibration dampers 19a is arranged on both sides of the second driver 16 in the z-direction. Not limited to this, only one vibration damper 19a may be arranged with respect to the second driver 16, or three or more vibration dampers 19a may be arranged. The vibration dampers 19a are arranged between the support 15 and the second driver 16 so as to fill gaps between the support 15 and the second driver 16 along the z-direction.

The vibration dampers 19a are arranged such that the vibration-damping surfaces 19a1 intersect a puncture operation direction of the needle 17a. In the present disclosure, the "puncture operation direction" corresponds to, for example, the z-direction. For example, the vibration-damping surfaces 19a1 are orthogonal to the z-direction, which is the puncture operation direction of the needle 17a. The vibration-damping surfaces 19a1 constitute contact surfaces with the first movable part 16b, in the vibration dampers 19a, and contact surfaces of the first movable part 16b in the z-direction. The vibration-damping surfaces 19a1 are included in the xy plane, as an example. When the second driver 16 vibrates and deforms the vibration dampers 19a by pressing the vibration dampers 19a, the vibration dampers 19a dampen the vibration by converting part of vibration energy of the second driver 16 into thermal energy.

The cell puncture device 10 has the regulator 19b that is arranged with respect to the second driver 16 and that regulates a movement direction of the second driver 16 to the puncture operation direction of the needle 17a. The regulator 19b includes a third fixed part 19b1 that is arranged with respect to the arm 14, and a second movable part 19b2 that is connected to the third fixed part 19b 1 and the second fixed part 16a of the second driver 16. The third fixed part 19b 1 is connected to, for example, an inner surface of the support 15 along the z-direction.

The regulator 19b includes, for example, a linear guide, a cross roller guide, or the like. The regulator 19b allows the second driver 16 to move in the puncture operation direction to cause the needle 17a to puncture the cell S, but regulates the movement of the second driver 16 in directions orthogonal to the puncture operation direction. For example, when the puncture operation direction is the z-direction, the regulator 19b allows the movement of the second driver 16 along the z-direction, but regulates the movement of the second driver 16 in the x- and y-directions.

The cell puncture device 10 may be configured so that the entire structure, which includes the first driver 13, the arm 14, the support 15, the second driver 16, and the needle 17a, can be evacuated from the microscope 20, by moving the pedestal 12 in the x- or y-direction relative to the first fixed part 11 or by rotatably moving the pedestal 12. The movement of the pedestal 12 in the x- or y-direction can be easily achieved by, for example, arranging a linear guide, a cross roller guide, or the like between the first fixed part 11 and the pedestal 12. The rotational movement of the pedestal 12 can be easily achieved by, for example, arranging a ball bearing, a cross roller bearing, or the like between the first fixed part 11 and the pedestal 12.

The cell puncture device 10 may further have a fixed part (not illustrated in the drawings) between the first fixed part 11 and the pedestal 12 in order to fix the relative movement of the pedestal 12 with respect to the first fixed part 11 in the x- or y-direction, or the relative rotational movement. For example, the fixed part may fix the pedestal 12 by a frictional force of a pin that can move in the z-direction and is pressed against the first fixed part 11 by a spring, or may fix the pedestal 12 by a similar pin engaging a groove provided in the first fixed part 11. The pin can be easily lifted up by arranging an operation unit to lift up the pin to the positive side in the z-direction. The pin allows the pedestal 12 to be moved and fixed relative to the first fixed part 11.

The cell puncture device 10 can accurately arrange the pedestal 12 with respect to the first fixed part 11, due to the above-described configuration. The cell puncture device 10 can reduce fluctuations in the position of the needle 17a due to play of the pedestal 12 with respect to the first fixed part 11.

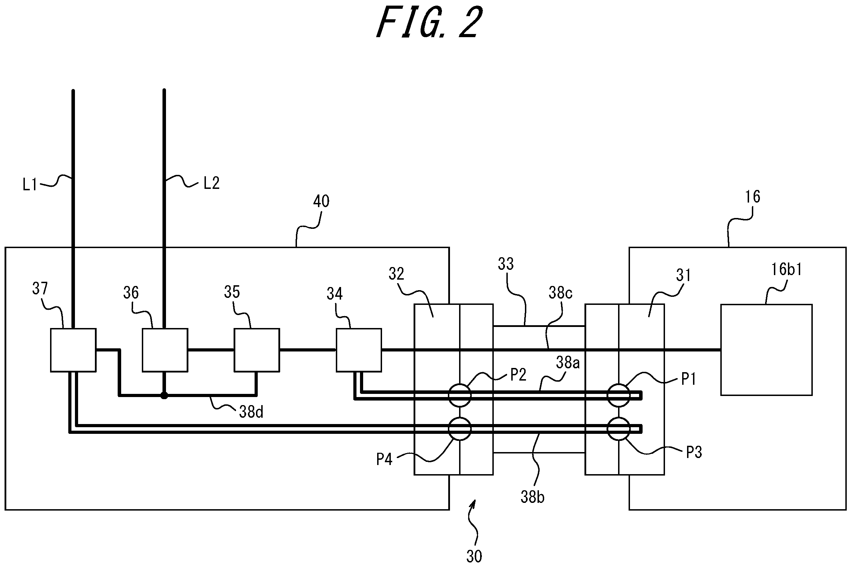

FIG. 2 is a schematic diagram illustrating an example configuration of the circuit module 30 according to the first embodiment of the present disclosure. The cell puncture device 10 further has the circuit module 30 illustrated in FIG. 2. The circuit module 30 outputs a voltage to a circuit element 16b1 contained in the second driver 16. The needle 17a of the needle unit 17, which is driven by the circuit element 16b1, punctures the cell S. The circuit element 16b1 includes a piezoelectric element, for example. For example, the second driver 16 needs to move the needle 17a at a high speed so that the needle 17a punctures the cell S, and the piezoelectric element may be used, as the circuit element 16b1, in order to achieve the high-speed movement of the needle 17a.

The circuit module 30 has a first connector 31 located on the circuit element 16b1's side and a second connector 32 located on the opposite side to the first connector 31. The circuit module 30 has a cable 33 that connects between the first connector 31 and the second connector 32. In FIG. 2, the first connector 31 is, as an example, directly attached to the second driver 16, which includes the circuit element 16b1, but is not limited to this. The first connector 31 may not be directly attached to the second driver 16, and may be attached to any of the other components of the cell puncture device 10 illustrated in FIG. 1. The second connector 32 may be, for example, directly attached to a controller 40 that controls the second driver 16.

The controller 40 and the second driver 16 are electrically connected to each other via the cable 33. The controller 40 controls the second driver 16 by being connected to the second driver 16 via the cable 33. For example, the controller 40 applies a voltage to the circuit element 16b1 contained in the second driver 16. The cell puncture device 10 further has the controller 40.

The controller 40 of the cell puncture device 10 is connected to a power line L1, which supplies power to the controller 40 from outside. The power line L1 is arranged to supply power to the controller 40 at a low voltage of 5 V, 24 V, or the like. The controller 40 is also connected to a signal line L2 in parallel with the power line L1. The signal line L2 is arranged to transmit, to the controller 40, a control signal necessary to execute control of the circuit element 16b1 from a control device at a higher level than the controller 40.

The circuit module 30 has a first breaker 34, a converter 35, a controller 36, and a second breaker 37. The first breaker 34, the converter 35, the controller 36, and the second breaker 37 are arranged in the controller 40 in this order from the second connector 32's side to the power line L1's side. The first breaker 34 includes, for example, a first switching element. The first switching element includes a relay, e.g., a semiconductor relay such as a photo MOS relay or a solid-state relay or a mechanical relay that switches connection using a mechanical contact, a switch, and the like. The converter 35 includes a conversion element that converts a low-voltage signal, which is input to the circuit module 30, into a high-voltage signal to drive the circuit element 16b1. The controller 36 includes a control element that outputs, to the converter 35, the control signal that is input as the low-voltage signal from the signal line L2. The second breaker 37 includes, for example, a second switching element. The second switching element includes a relay, e.g., a semiconductor relay such as a photo MOS relay or a solid-state relay or a mechanical relay that switches connection using a mechanical contact, a switch, and the like.

The circuit module 30 has a first checker that changes a status when the first or second connector 31 or 32 and the cable 33 are in a disconnected state. In the first embodiment, the first checker includes first wiring 38a that loops between the first breaker 34 and the first connector 31 via the second connector 32 and the cable 33. In the first embodiment, the "status" of the first checker includes, for example, the conduction status of the first wiring 38a as the first checker, i.e., the connection status of the loop.

The first wiring 38a extends from the first breaker 34 and passes through a second node P2 between the second connector 32 and the cable 33. The first wiring 38a further extends inside the cable 33 and reaches a first node P1 between the first connector 31 and the cable 33. The first wiring 38a turns back inside the first connector 31, passes through the first node P1, extends inside the cable 33, and returns to the first breaker 34 via the second node P2. The first wiring 38a is not limited to the structure of turning back inside the first connector 31, but may turn back outside the first connector 31.

The circuit module 30 has a second checker that changes a status when the first or second connector 31 or 32 and the cable 33 are in a disconnected state. In the first embodiment, the second checker includes second wiring 38b that loops between the second breaker 37 and the first connector 31 via the second connector 32 and the cable 33. In the first embodiment, the "status" of the second checker includes, for example, the conduction status of the second wiring 38b as the second checker, i.e., the connection status of the loop.

The second wiring 38b extends from the second breaker 37 and passes through a fourth node P4 between the second connector 32 and the cable 33. The second wiring 38b further extends inside the cable 33 and reaches a third node P3 between the first connector 31 and the cable 33. The second wiring 38b turns back inside the first connector 31, passes through the third node P3, extends inside the cable 33, and returns to the second breaker 37 via the fourth node P4. The second wiring 38b is not limited to the structure of turning back inside the first connector 31, but may turn back outside the first connector 31.

Each of the first node P1, the second node P2, the third node P3, and the fourth node P4 may be, for example, nodes constituted of two pairs of male and female connectors that form the loop, and may be connector terminals constituted of two pairs of plugs and receptacles.

The circuit module 30 has third wiring 38c that extends from the controller 36 to the circuit element 16b1 via the converter 35, the first breaker 34, the second connector 32, the cable 33, and the first connector 31. The third wiring 38c is arranged to transmit, from the controller 36 to the circuit element 16b1, the control signal from the signal line L2. The circuit module 30 has fourth wiring 38d that is connected from the second breaker 37 to both the controller 36 and the converter 35.

As described above, the cable 33 integrates the first wiring 38a as the first checker and the second wiring 38b as the second checker, in addition to the third wiring 38c as a control line for controlling the circuit element 16b1. The cable 33 connects between the second driver 16, which contains the circuit element 16b1, and the controller 40 by being connected to the first connector 31 at one end and to the second connector 32 at the other end.

The functions of each component of the circuit module 30 when the cable 33 is connected to both the first connector 31 and the second connector 32 will be mainly described.

The second breaker 37 supplies power, which is input from the power line L1, to the controller 36 and the converter 35 via the fourth wiring 38d. The controller 36 outputs the control signal, which is input from the signal line L2 as the low-voltage signal, to the converter 35 as a signal to operate the circuit element 16b1. At this time, the controller 36 may convert the signal format of the control signal, as necessary. The converter 35 converts the control signal, which is output from the controller 36 as the low-voltage signal, into the high-voltage signal to drive the circuit element 16b1. The first breaker 34 passes the high-voltage signal, which is output from the converter 35. The third wiring 38c transmits the high-voltage signal, which has passed through the first breaker 34, to the circuit element 16b1. This causes the circuit element 16b1 to operate.

Next, the functions of the first checker and the first breaker 34, and the second checker and the second breaker 37 will be mainly described.

The first breaker 34 is connected to the converter 35 via the third wiring 38c, and is also connected to the first wiring 38a as the first checker. The first breaker 34 contributes a break of the voltage to the circuit element 16b1 in response to a change in the status of the first checker. For example, the first breaker 34 breaks the high-voltage signal, which is output from the converter 35, with being arranged between the converter 35 and the second connector 32. In this disclosure, "arranged between A and B" means that being able to be arranged at any position between A and B.

For example, when the cable 33 is connected to both the first connector 31 and the second connector 32, and the loop of the first wiring 38a, as the first checker, is in a connected state, the first breaker 34 is turned on and transmits the high-voltage signal, which is output from the converter 35. On the other hand, when the cable 33 is not connected to at least one of the first connector 31 or the second connector 32, and the loop of the first wiring 38a, as the first checker, is in a disconnected state, the first breaker 34 is turned off and breaks the high-voltage signal, which is output from the converter 35. For example, when the cable 33 is not connected to the first connector 31, the loop of the first wiring 38a breaks at the first node P1. For example, when the cable 33 is not connected to the second connector 32, the loop of the first wiring 38a breaks at the second node P2.

The second breaker 37 is connected to the power line L1, the fourth wiring 38d, and the second wiring 38b. The second breaker 37 contributes a break of the voltage to the circuit element 16b1 in response to a change in the status of the second checker. For example, the second breaker 37 breaks power supply to the converter 35, with being arranged between the power line L1, which is from outside the circuit module 30, and the converter 35.

For example, when the cable 33 is connected to both the first connector 31 and the second connector 32, and the loop of the second wiring 38b, as the second checker, is in a connected state, the second breaker 37 is turned on and maintains the power supply to the converter 35. On the other hand, when the cable 33 is not connected to at least one of the first connector 31 or the second connector 32, and the loop of the second wiring 38b, as the second checker, is in a disconnected state, the second breaker 37 is turned off and breaks the power supply to the converter 35. For example, when the cable 33 is not connected to the first connector 31, the loop of the second wiring 38b breaks at the third node P3. For example, when the cable 33 is not connected to the second connector 32, the loop of the second wiring 38b breaks at the fourth node P4.

The circuit module 30 according to the first embodiment described above improves safety in supplying the voltage to the circuit element 16b1. Due to the provision of the first checker and the first breaker 34, the circuit module 30 can contribute the break of the voltage to the circuit element 16b1 at a single point when the cable 33 is not connected to at least one of the first connector 31 or the second connector 32. Due to the provision of the second checker and the second breaker 37, the circuit module 30 can contribute the break of the voltage to the circuit element 16b1 at another point when the cable 33 is not connected to at least one of the first connector 31 or the second connector 32. As a result, the circuit module 30 can contribute the break of the voltage at multiple points when the cable 33 is in the disconnected state, thus allowing more reliable suppression of output of the voltage.

The first checker includes the first wiring 38a, which loops between the first breaker 34 and the first connector 31 via the second connector 32 and the cable 33. This allows the circuit module 30 to directly break the voltage to the circuit element 16b1 in response to a change in the conduction status of the first wiring 38a. Therefore, when the cable 33 is in the disconnected state, the circuit module 30 can suppress output of the voltage more reliably.

The second checker includes the second wiring 38b, which loops between the second breaker 37 and the first connector 31 via the second connector 32 and the cable 33. This allows the circuit module 30 to indirectly break the voltage to the circuit element 16b1 in response to a change in the conduction status of the second wiring 38b. Therefore, when the cable 33 is in the disconnected state, the circuit module 30 can suppress output of the voltage more reliably.

The circuit module 30 has the converter 35 that is arranged on the second connector 32's side and that converts the low-voltage signal, which is input to the circuit module 30, into the high-voltage signal to drive the circuit element 16b1. Therefore, the circuit module 30 can easily drive the circuit element 16b1 even when a high voltage is required to drive the circuit element 16b1.

The first breaker 34 is arranged between the converter 35 and the second connector 32, and breaks the high-voltage signal, which is output from the converter 35. Therefore, the circuit module 30 can directly break, using the first breaker 34, the voltage to the circuit element 16b1.

The second breaker 37 is arranged between the power line L1, which is from outside the circuit module 30, and the converter 35, and breaks the power supply to the converter 35. Therefore, the circuit module 30 can indirectly break, using the second breaker 37, the voltage to the circuit element 16b1.

Since the circuit element 16b1 includes the piezoelectric element, the cell puncture device 10 can move the needle 17a at a high speed when the needle 17a punctures the cell S. Therefore, in order to inject a chemical solution into the cell S or to take out a substance inside the cell S by suction, the cell puncture device 10 can easily control the position and speed of the needle tip so that the needle tip penetrates a cell wall. By combining the circuit module 30 and the piezoelectric element, the cell puncture device 10 can improve safety even in the applied voltage to the piezoelectric element, which may be as high as 150 V, for example.

In the first embodiment described above, the circuit module 30 is described as having the converter 35 that is arranged on the second connector 32's side and that converts the low-voltage signal, which is input to the circuit module 30, into the high-voltage signal to drive the circuit element 16b1, but is not limited to this. The circuit module 30 may not have the converter 35.

In the first embodiment described above, the first breaker 34 is described as being arranged between the converter 35 and the second connector 32, and as breaking the high-voltage signal, which is output from the converter 35, but is not limited to this. The first breaker 34 may be arranged at another position in the circuit module 30, and break the circuit at that position. For example, the first breaker 34 may be arranged immediately after the controller 36, and break the low-voltage signal, which is output from the controller 36.

In the first embodiment described above, the second breaker 37 is described as being arranged between the power line L1, which is from outside the circuit module 30, and the converter 35, and as breaking the power supply to the converter 35, but is not limited to this. The second breaker 37 may be arranged at another position in the circuit module 30, and break the circuit at that position. As illustrated in FIG. 2, the second breaker 37 may break power supply to the controller 36, instead of or in addition to the power supply to the converter 35, with being connected to the controller 36 in addition to the converter 35.

In the first embodiment described above, the circuit element 16b1 is described as including the piezoelectric element, but is not limited to this. The circuit element 16b1 may include any other element that operates with application of a voltage from the circuit module 30.

In the first embodiment described above, the circuit module 30 is described as being used in the cell puncture device 10 and the microscope system 1, but is not limited to this. The circuit module 30 may be used in any other device that has a circuit element to which a voltage is to be output.

In the first embodiment described above, for example, the first fixed part 11 is described as being fixed to the microscope 20 that images the cell S, but is not limited to this. The first fixed part 11 may be attached to the microscope 20 in another aspect, as long as the first fixed part 11 is arranged with respect to the imaging unit that images the cell S. For example, the first fixed part 11 may be incorporated inside the microscope 20, instead of being fixed so as to extend outside the microscope 20 as described above.

### (Second Embodiment)

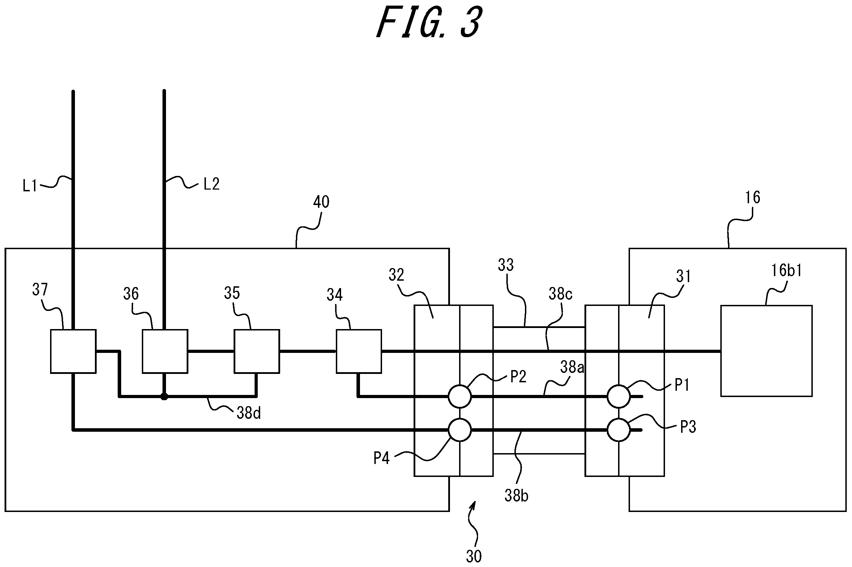

FIG. 3 is a schematic diagram illustrating an example configuration of a circuit module 30 according to a second embodiment of the present disclosure. An example of the configuration and functions of the circuit module 30 according to the second embodiment will be mainly described with reference to FIG. 3.

In the first embodiment described above, the first checker is described as including the first wiring 38a that loops between the first breaker 34 and the first connector 31 via the second connector 32 and the cable 33, but is not limited to this. The first checker may include multiple communication elements described below. Similarly, in the first embodiment described above, the second checker is described as including the second wiring 38b that loops between the second breaker 37 and the first connector 31 via the second connector 32 and the cable 33, but is not limited to this. The second checker may include multiple communication elements described below.

The circuit module 30 according to the second embodiment differs from that in the first embodiment in the points described above. The other components, functions, effects, variations, and the like are the same as those in the first embodiment, and the corresponding descriptions also apply to the circuit module 30 according to the second embodiment. In the following, the same components as in the first embodiment are indicated with the same reference numerals, and descriptions thereof are omitted. The points that differ from the first embodiment will be mainly described.

As illustrated in FIG. 3, first wiring 38a extends from a first breaker 34 and passes through a second node P2 between a second connector 32 and a cable 33. The first wiring 38a further extends inside the cable 33, and reaches a first node P1 between a first connector 31 and the cable 33. In the second embodiment, the first wiring 38a may be arranged with its end being broken inside the first connector 31. The first wiring 38a may have a termination inside the first connector 31 without looping.

In the second embodiment, a first checker may include multiple communication elements, instead of the first wiring 38a. For example, the first checker may include a first communication element that is arranged at the first node P1 between the cable 33 and the first connector 31, and that detects a disconnected state between the first connector 31 and the cable 33 and outputs a first communication signal to the first breaker 34. The first checker may include a second communication element that is arranged at the second node P2 between the cable 33 and the second connector 32, and that detects a disconnected state between the second connector 32 and the cable 33 and outputs a second communication signal to the first breaker 34. In the second embodiment, the "status" of the first checker may include, for example, each of the output status of the first communication signal from the first communication element as the first checker and the output status of the second communication signal from the second communication element as the first checker.

The first communication element may include, for example, a communication element that enables communication via controller area network (CAN), a communication element that enables communication via recommended standard 232 version C (RS-232C), a communication element that enables wireless communication via radio frequency identification (RF-ID), or the like. The first communication element may output, to the first breaker 34, the first communication signal indicating the disconnected state when the first connector 31 and the cable 33 are not connected to each other at the first node P1, through a wire using the first wiring 38a, or wirelessly without using the first wiring 38a.

The second communication element may include, for example, a communication element that enables communication via CAN, a communication element that enables communication via RS-232C, a communication element that enables wireless communication via RF-ID, or the like. The second communication element may output, to the first breaker 34, the second communication signal indicating the disconnected state when the second connector 32 and the cable 33 are not connected to each other at the second node P2, through a wire using the first wiring 38a, or wirelessly without using the first wiring 38a.

The first breaker 34 contributes a break of voltage to a circuit element 16b1 in response to a change in the status of the first checker. For example, the first breaker 34 breaks a high-voltage signal, which is output from a converter 35, with being arranged between the converter 35 and the second connector 32. For example, upon acquiring at least one of the first communication signal from the first communication element or the second communication signal from the second communication element, the first breaker 34 may be turned off and break the high-voltage signal, which is output from the converter 35.

As illustrated in FIG. 3, second wiring 38b extends from a second breaker 37 and passes through a fourth node P4 between the second connector 32 and the cable 33. The second wiring 38b further extends inside the cable 33, and reaches a third node P3 between the first connector 31 and the cable 33. In the second embodiment, the second wiring 38b may be arranged with its end being broken inside the first connector 31. The second wiring 38b may have a termination inside the first connector 31 without looping.

In the second embodiment, a second checker may include multiple communication elements, instead of the second wiring 38b. For example, the second checker may include a third communication element that is arranged at the third node P3 between the cable 33 and the first connector 31, and that detects a disconnected state between the first connector 31 and the cable 33 and outputs a third communication signal to the second breaker 37. The second checker may include a fourth communication element that is arranged at the fourth node P4 between the cable 33 and the second connector 32, and that detects a disconnected state between the second connector 32 and the cable 33 and outputs a fourth communication signal to the second breaker 37. In the second embodiment, the "status" of the second checker may include, for example, each of the output status of the third communication signal from the third communication element as the second checker and the output status of the fourth communication signal from the fourth communication element as the second checker.

The third communication element may include, for example, a communication element that enables communication via CAN, a communication element that enables communication via RS-232C, a communication element that enables wireless communication via RF-ID, or the like. The third communication element may output, to the second breaker 37, the third communication signal indicating the disconnected state when the first connector 31 and the cable 33 are not connected to each other at the third node P3, through a wire using the second wiring 38b, or wirelessly without using the second wiring 38b.

The fourth communication element may include, for example, a communication element that enables communication via CAN, a communication element that enables communication via RS-232C, a communication element that enables wireless communication via RF-ID, or the like. The fourth communication element may output, to the second breaker 37, the fourth communication signal indicating the disconnected state when the second connector 32 and the cable 33 are not connected to each other at the fourth node P4, through a wire using the second wiring 38b, or wirelessly without using the second wiring 38b.

The second breaker 37 contributes a break of voltage to the circuit element 16b1 in response to a change in the status of the second checker. For example, the second breaker 37 breaks power supply to the converter 35, with being arranged between a power line L1, which is from outside the circuit module 30, and the converter 35. For example, upon acquiring at least one of the third communication signal from the third communication element or the fourth communication signal from the fourth communication element, the second breaker 37 may be turned off and break the power supply to the converter 35.

The circuit module 30 according to the second embodiment described above has the same effects as the first embodiment. In addition, since the first checker includes the first communication element and the second communication element, the circuit module 30 can directly break the voltage to the circuit element 16b1 in response to a change in the output status of the communication signal from each communication element. Therefore, when the cable 33 is in the disconnected state, the circuit module 30 can suppress output of the voltage more reliably.

Since the second checker includes the third communication element and the fourth communication element, the circuit module 30 can indirectly break the voltage to the circuit element 16b1 in response to a change in the output status of the communication signal from each communication element. Therefore, when the cable 33 is in the disconnected state, the circuit module 30 can suppress output of the voltage more reliably.

It is obvious to those skilled in the art that the present disclosure can be realized in other predetermined forms excluding the above-described embodiments, without departing from the spirit or the essential features thereof. Therefore, the foregoing descriptions are illustrative and not limited thereto. The scope of the disclosure is defined by the appended claims, not by the foregoing descriptions. Among any changes, some changes within the scope of equivalence thereof shall be included therein.

For example, the shapes, patterns, sizes, arrangements, orientations, types, numbers, and the like of the components described above are not limited to the contents of the descriptions and drawings above. The shapes, patterns, sizes, arrangements, orientations, types, numbers, and the like of the components may be configured arbitrarily as long as the functions thereof can be achieved. The illustrated components of the circuit module 30, the cell puncture device 10, and the microscope system 1 are conceptual in terms of functions, and the specific forms of the components are not limited to those illustrated.

The functions and the like included in each of the above-described components and the like can be rearranged so that there are no logical contradictions, and it is possible to combine multiple components or the like into one or split one component into multiple.

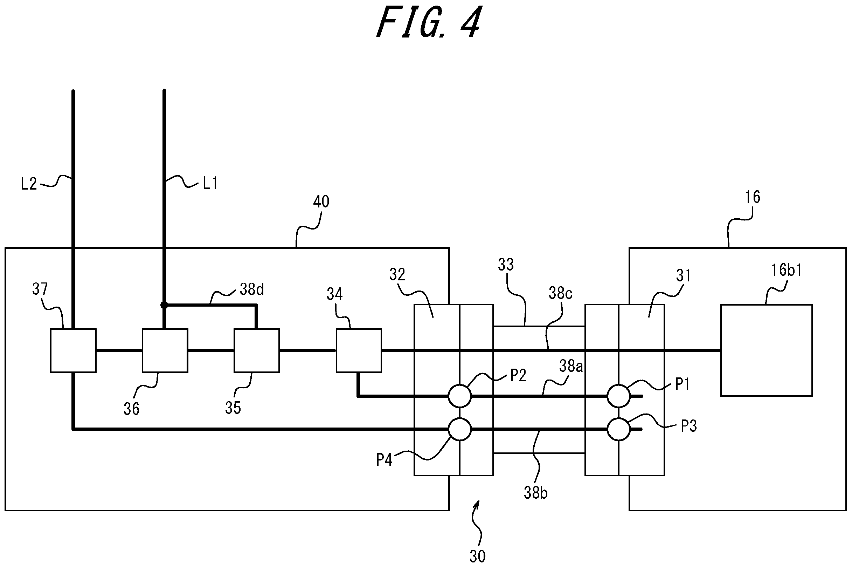

FIG. 4 is a schematic diagram illustrating an example configuration of a circuit module 30 according to a first variation of the present disclosure. An example of the configuration and functions of the circuit module 30 according to the first variation will be mainly described with reference to FIG. 4. In FIG. 4, a difference regarding the variation is illustrated so as to correspond to FIG. 3, which illustrates the circuit module 30 according to the second embodiment, but this difference also applies to the circuit module 30 according to the first embodiment.

In the above first and second embodiments, the second breaker 37 is described as being arranged between the power line L1, which is from outside the circuit module 30, and the converter 35, and as breaking the power supply to the converter 35, but is not limited to this. As illustrated in FIG. 4, the second breaker 37 may be arranged between the signal line L2, which is from outside the circuit module 30, and the converter 35, and break the low-voltage signal to be input to the converter 35.

For example, upon acquiring at least one of the third communication signal from the third communication element or the fourth communication signal from the fourth communication element, the second breaker 37 may be turned off and break the low-voltage signal to be input to the converter 35. For example, the second breaker 37 does not output, to the controller 36, the low-voltage signal that is input from the signal line L2. As a result, the low-voltage signal is not output from the controller 36 to the converter 35, and therefore no high-voltage signal is generated.

In the first variation illustrated in FIG. 4, the second breaker 37 is connected to the signal line L2 and the second wiring 38b. In addition, the second breaker 37 is also connected to the third wiring 38c. In the controller 40, the second breaker 37, the controller 36, the converter 35, and the first breaker 34 are arranged in this order on the third wiring 38c from the signal line L2 to the second connector 32.

The circuit module 30 according to the first variation described above can indirectly break, using the second breaker 37, the voltage to the circuit element 16b1. The circuit module 30 also has the same effects as the first and second embodiments. In other words, the circuit module 30 can suppress output of the high-voltage signal more reliably by performing double breaks using the second breaker 37 and the first breaker 34 when the cable 33 is in the disconnected state, and therefore improve safety.

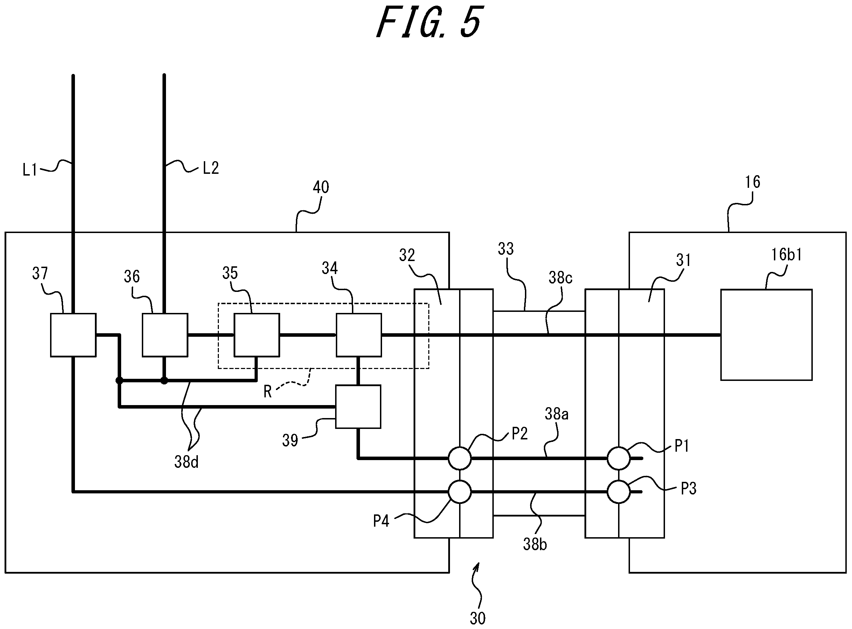

FIG. 5 is a schematic diagram illustrating an example configuration of a circuit module 30 according to a second variation of the present disclosure. An example of the configuration and functions of the circuit module 30 according to the second variation will be mainly described with reference to FIG. 5. In FIG. 5, a difference regarding the variation is illustrated so as to correspond to FIG. 3, which illustrates the circuit module 30 according to the second embodiment, but this difference also applies to the circuit modules 30 according to the first embodiment and the first variation.

In the above first and second embodiments, the circuit module 30 is described as having only two breakers, i.e., the first breaker 34 and the second breaker 37, but is not limited to this. The circuit module 30 may have three or more breakers. For example, as illustrated in FIG. 5, the circuit module 30 may further have a third breaker 39 that contributes a break of voltage in response to a change in the status of the first checker. The third breaker 39 may be arranged between the power line L1, which is from outside the circuit module 30, and the first breaker 34, and break power supply to the first breaker 34 in order to break the first breaker 34 or cancel the break of the first breaker 34.

As illustrated by the broken line in FIG. 5, in the controller 40, a high-voltage area R includes the converter 35 and the first breaker 34. The high-voltage area R is, for example, an area that includes the surroundings of the third wiring 38c, to which a high voltage is applied by the converter 35, and an area that ensures a space distance and a creepage distance that do not discharge depending on a maximum voltage applied. In other words, the high-voltage area R is an area in which, when an unintended conductor enters the high-voltage area R, a high voltage may be conducted due to discharge from the third wiring 38c with the high voltage. The high-voltage area R is an area that is preferably protected to prevent unintended conductors from entering. The area other than the high-voltage area R, which is enclosed by the broken line, in FIG. 5 is a low-voltage area.

The third breaker 39 is arranged in the low-voltage area on the first wiring 38a. The third breaker 39 is connected to the first breaker 34 via the first wiring 38a. When the cable 33 is in the connected state, the third breaker 39 transmits power, which has been input from the power line L1 via the second breaker 37 and the fourth wiring 38d, to the first breaker 34 to perform power supply. The first breaker 34 receives the power supply from the third breaker 39, and outputs the high-voltage signal, which is output from the converter 35, to the circuit element 16b1 via the third wiring 38c.

The third breaker 39 includes a relay element, for example, and outputs a power of, e.g., +5 V to the first breaker 34, when there is power supply in conjunction with the first checker. Upon receiving the power from the third breaker 39, the first breaker 34 outputs the high-voltage signal, which is input from the converter 35, without breaking. On the other hand, the third breaker 39 does not output the power of, e.g., +5 V to the first breaker 34 when there is no power supply in conjunction with the first checker. Upon receiving no power from the third breaker 39, the first breaker 34 breaks the high-voltage signal, which is input from the converter 35.

The circuit module 30 according to the second variation described above can indirectly break, using the third breaker 39, the voltage to the circuit element 16b1. The circuit module 30 further has the third breaker 39, as compared to the first and second embodiments, and therefore break the voltage using the three breakers when the cable 33 is in the disconnected state. The circuit module 30 can perform double breaks using the second breaker 37 and the third breaker 39 at the low-voltage side to the first breaker 34 that needs to be selected as one that can be used at a high voltage. Therefore, the circuit module 30 can suppress output of the high-voltage signal more reliably, and improve safety.

Examples of some embodiments of the present disclosure are described below. However, it should be noted that the embodiments of the present disclosure are not limited to these examples.
[Appendix 1] A circuit module configured to output a voltage to a circuit element, the circuit module comprising:
   a first connector arranged on the circuit element's side;
   a second connector arranged on an opposite side to the first connector;
   a cable connecting between the first connector and the second connector;
   a first checker configured to change a status when the first or second connector and the cable are in a disconnected state;
   a second checker configured to change a status when the first or second connector and the cable are in a disconnected state;
   a first breaker configured to contribute a break of the voltage in response to a change in the status of the first checker; and
   a second breaker configured to contribute a break of the voltage in response to a change in the status of the second checker.
[Appendix 2] The circuit module according to appendix 1, wherein the first checker includes first wiring that loops between the first breaker and the first connector via the second connector and the cable.
[Appendix 3] The circuit module according to appendix 1 or 2, wherein the second checker includes second wiring that loops between the second breaker and the first connector via the second connector and the cable.
[Appendix 4] The circuit module according to any one of appendices 1 to 3, wherein the first checker includes:
   a first communication element arranged at a first node between the cable and the first connector, the first communication element being configured to detect the disconnected state between the first connector and the cable and output a first communication signal to the first breaker; and
   a second communication element arranged at a second node between the cable and the second connector, the second communication element being configured to detect the disconnected state between the second connector and the cable and output a second communication signal to the first breaker.
[Appendix 5] The circuit module according to any one of appendices 1 to 4, wherein the second checker includes:
   a third communication element arranged at a third node between the cable and the first connector, the third communication element being configured to detect the disconnected state between the first connector and the cable and output a third communication signal to the second breaker; and
   a fourth communication element arranged at a fourth node between the cable and the second connector, the fourth communication element being configured to detect the disconnected state between the second connector and the cable and output a fourth communication signal to the second breaker.
[Appendix 6] The circuit module according to any one of appendices 1 to 5, further comprising a converter arranged on the second connector's side, the converter configured to convert a low-voltage signal that is input to the circuit module into a high-voltage signal to drive the circuit element.
[Appendix 7] The circuit module according to appendix 6, wherein the first breaker is arranged between the converter and the second connector, and is configured to break the high-voltage signal that is output from the converter.
[Appendix 8] The circuit module according to appendix 6 or 7, wherein the second breaker is arranged between a power line from outside the circuit module and the converter, and is configured to break power supply to the converter.
[Appendix 9] The circuit module according to any one of appendices 6 to 8, wherein the second breaker is arranged between a signal line from outside the circuit module and the converter, and is configured to break the low-voltage signal to be input to the converter.
[Appendix 10] The circuit module according to any one of appendices 1 to 9, further comprising:
   a third breaker configured to contribute a break of the voltage in response to a change in the status of the first checker, and
   wherein the third breaker is arranged between a power line from outside the circuit module and the first breaker, and is configured to break power supply to the first breaker, in order to break the first breaker or cancel a break of the first breaker.
[Appendix 11] A cell puncture device comprising:
   the circuit module according to any one of appendices 1 to 10;
   the circuit element; and
   a needle configured to be driven by the circuit element, the needle configured to puncture a cell.
[Appendix 12] The cell puncture device according to appendix 11, wherein the circuit element includes a piezoelectric element.
[Appendix 13] A microscope system comprising the cell puncture device according to appendix 11 or 12.

## Claims

1. A circuit module configured to output a voltage to a circuit element, the circuit module comprising:
a first connector arranged on the circuit element's side;
a second connector arranged on an opposite side to the first connector;
a cable connecting between the first connector and the second connector;
a first checker configured to change a status when the first or second connector and the cable are in a disconnected state;
a second checker configured to change a status when the first or second connector and the cable are in a disconnected state;
a first breaker configured to contribute a break of the voltage in response to a change in the status of the first checker; and
a second breaker configured to contribute a break of the voltage in response to a change in the status of the second checker.

2. The circuit module according to claim 1, wherein the first checker includes first wiring that loops between the first breaker and the first connector via the second connector and the cable.

3. The circuit module according to claim 1 or 2, wherein the second checker includes second wiring that loops between the second breaker and the first connector via the second connector and the cable.

4. The circuit module according to claim 1 or 2, wherein the first checker includes:
a first communication element arranged at a first node between the cable and the first connector, the first communication element being configured to detect the disconnected state between the first connector and the cable and output a first communication signal to the first breaker; and
a second communication element arranged at a second node between the cable and the second connector, the second communication element being configured to detect the disconnected state between the second connector and the cable and output a second communication signal to the first breaker.

5. The circuit module according to claim 1 or 2, wherein the second checker includes:
a third communication element arranged at a third node between the cable and the first connector, the third communication element being configured to detect the disconnected state between the first connector and the cable and output a third communication signal to the second breaker; and
a fourth communication element arranged at a fourth node between the cable and the second connector, the fourth communication element being configured to detect the disconnected state between the second connector and the cable and output a fourth communication signal to the second breaker.

6. The circuit module according to claim 1 or 2, further comprising a converter arranged on the second connector's side, the converter configured to convert a low-voltage signal that is input to the circuit module into a high-voltage signal to drive the circuit element.

7. The circuit module according to claim 6, wherein the first breaker is arranged between the converter and the second connector, and is configured to break the high-voltage signal that is output from the converter.

8. The circuit module according to claim 6, wherein the second breaker is arranged between a power line from outside the circuit module and the converter, and is configured to break power supply to the converter.

9. The circuit module according to claim 6, wherein the second breaker is arranged between a signal line from outside the circuit module and the converter, and is configured to break the low-voltage signal to be input to the converter.

10. The circuit module according to claim 1 or 2, further comprising:
a third breaker configured to contribute a break of the voltage in response to a change in the status of the first checker, and
wherein the third breaker is arranged between a power line from outside the circuit module and the first breaker, and is configured to break power supply to the first breaker, in order to break the first breaker or cancel a break of the first breaker.

11. A cell puncture device comprising:
the circuit module according to claim 1 or 2;
the circuit element; and
a needle configured to be driven by the circuit element, the needle configured to puncture a cell.

12. The cell puncture device according to claim 11, wherein the circuit element includes a piezoelectric element.

13. A microscope system comprising the cell puncture device according to claim 11.
